# DEMANDE DE BREVET EUROPEEN

(11) **EP 4 656 136 A1**
(43) Date de publication de la demande: **03.12.2025**
(21) Numéro de dépôt: 25177767.8
(22) Date de dépôt: 20.05.2025
(51) Int. Cl.: A61B 5/20, A61B 5/00, A61B 10/00, G01N 33/493, A61B 5/145, E03D 9/03

(54) **NETTOYAGE D'UN DISPOSITIF D'ANALYSE D'URINE**

(30) Priorité: 30.05.2024 FR 2405625
(71) Demandeur: Withings, 92130 Issy-Les-Moulineaux (FR)
(72) Inventeur: Pons, Arthur, 92130 Issy-les-Moulineaux (FR)
(74) Mandataire: Withings IP

(57) **Abrégé**

L'invention concerne un ensemble pour analyse d'urine comprenant :
- un dispositif d'analyse d'urine (100) configuré pour être placé entièrement dans une cuvette (106) de toilettes et comprenant un boîtier (204), le boîtier (204) comprenant une face avant (220) destinée à recevoir un flux d'urine provenant directement d'un utilisateur urinant dans la cuvette (106) des toilettes,
- un bras de fixation (500) configuré pour positionner le dispositif d'analyse d'urine (100) entièrement dans la cuvette (106) de toilettes, le bras de fixation (500) comprenant une rampe (560) d'amenée d'eau configurée pour orienter un flux d'eau (F) provenant d'une chasse d'eau (670) de la cuvette (106) de toilettes vers la face avant (220) du boîtier (204).

## Description

La présente invention concerne un ensemble pour analyse d'urine comprenant un dispositif d'analyse d'urine. Le dispositif d'analyse d'urine comprend un boîtier configuré pour être placé entièrement dans une cuvette de toilettes.

### Etat de la technique

De nombreux paramètres biologiques sont identifiables dans l'urine d'un individu. Par exemple, des problèmes de santé tels qu'une infection des voies urinaires, un diabète ou une insuffisance rénale peuvent être détectés à partir d'un échantillon d'urine. L'échantillon d'urine peut également refléter la qualité d'un régime alimentaire, identifier une période de fertilité ou de grossesse et détecter la consommation de drogues ou de tabac. Il est donc intéressant de surveiller périodiquement divers paramètres biologiques.

Il est connu de proposer des dispositifs de toilette avec une fonction d'analyse d'urine. Ces dispositifs sont capables de prélever des échantillons d'urine dans les toilettes et de les analyser pour déterminer le niveau d'un paramètre biologique.

Un tel dispositif d'analyse d'urine est notamment décrit dans le document WO2021/175909. Ce document divulgue un dispositif pour l'analyse d'urine. Le dispositif se loge dans les toilettes et recueille un échantillon d'urine avant d'effectuer une analyse optique. Le dispositif comprend une station et une cartouche, également appelée support rotatif, qui peut être retirée et remplacée de la station. La cartouche contient des bandelettes urinaires, c'est-à-dire des bandelettes enduites ou imprégnées d'un réactif réagissant avec l'urine.

Un tel dispositif d'analyse d'urine placé dans une cuvette de toilettes fait face à une problématique due au nettoyage du boitier après qu'un utilisateur ait uriné dessus. En effet, la cuvette de toilettes comprend une chasse d'eau qui fait couler de l'eau de long de la cuvette. Ce flux d'eau a tendance à passer au-dessus et/ou derrière le boîtier et ne nettoie donc pas la face avant du boitier, ce qui peut présenter des problématiques de confort et d'hygiène (couleur jaunie du boitier, odeur, etc.) pour l'utilisateur.

### Résumé de l'invention

La présente description propose donc un ensemble d'analyse d'urine permettant un nettoyage du dispositif d'analyse d'urine après une miction d'un utilisateur.

A cet effet, la présente description concerne un ensemble pour analyse d'urine comprenant :
- un dispositif d'analyse d'urine configuré pour être placé entièrement dans une cuvette de toilettes et comprenant un boîtier, le boîtier comprenant une face avant destinée à recevoir un flux d'urine provenant directement d'un utilisateur urinant dans la cuvette des toilettes,
- un bras de fixation configuré pour positionner le dispositif d'analyse d'urine entièrement dans la cuvette de toilettes, le bras de fixation comprenant une rampe d'amenée d'eau configurée pour orienter un flux d'eau provenant d'une chasse d'eau de la cuvette de toilettes vers la face avant du boîtier.

En effet, un tel ensemble d'analyse d'urine permet de fournir un dispositif d'analyse d'urine disposé dans des toilettes, et donc subissant de nombreuses mictions d'utilisateur sur la face avant, pouvant être nettoyé au moins en partie au moyen du bras de fixation. En effet, la rampe d'amenée d'eau du bras de fixation permet d'orienter un flux d'eau de la chasse d'eau vers la face avant du boitier et ainsi nettoyer en partie cette face avant.

Dans un mode de réalisation, le bras de fixation est flexible.

Dans un mode de réalisation, le bras de fixation est amovible.

Dans un mode de réalisation, le bras de fixation présente une forme aplatie dans une section transverse à un plan médian du dispositif.

Dans un mode de réalisation, le bras de fixation présente une largeur comprise entre 0,5 cm et 20 cm.

Dans un mode de réalisation, la rampe s'étend sur une longueur comprise entre 1cm et 20 cm.

Dans un mode de réalisation, la rampe s'étend dans le prolongement de la face avant du boîtier du dispositif d'analyse d'urine ou est en surplomb de la face avant.

Dans un mode de réalisation, la rampe affleure la face avant.

Dans un mode de réalisation, la rampe s'étend depuis le boîtier en direction de la cuvette, et forme un angle compris entre 0° et 90° avec un plan tangent à la face avant, notamment entre 30° et 60°.

Dans un mode de réalisation, la rampe est convexe longitudinalement et forme un tremplin pour l'eau.

Dans un mode de réalisation, la rampe est convexe transversalement et forme une rigole pour l'eau.

Dans un mode de réalisation, la rampe présente au moins une ailette configurée pour s'étendre en direction de la cuvette des toilettes.

Dans un mode de réalisation, la rampe présente des ailettes latérales.

Dans un mode de réalisation, la rampe comprend un diffuseur pour l'eau de la chasse d'eau, le diffuseur étant configuré pour disperser l'eau du flux d'eau de la rampe sur la face avant du boîtier.

Dans un mode de réalisation, le diffuseur est positionné sur une section de transvasement au niveau de la jonction avec le boitier.

Dans un mode de réalisation, le diffuseur comprend des rainures.

Dans un mode de réalisation, le bras de fixation comprend une portion d'extrémité proximale configurée pour coopérer avec le boîtier.

Dans un mode de réalisation, le bras de fixation comprend en outre une portion d'extrémité distale configurée pour coopérer avec la cuvette de toilettes.

Dans un mode de réalisation, le bras de fixation comprend en outre une portion intermédiaire arrangée entre la portion d'extrémité proximale et la portion d'extrémité distale.

Dans un mode de réalisation, la portion d'extrémité distale comprend un crochet/un aimant/une partie adhésive.

Dans un mode de réalisation, la rampe comprend une section de transvasement à proximité du boitier et configurée pour favoriser le passage de l'eau du flux d'eau de la rampe vers la face avant du boîtier.

Dans un mode de réalisation, la section de transvasement affleure la face avant du boîtier.

Dans un mode de réalisation, le diffuseur est positionné au niveau de la section de transvasement.

Dans un mode de réalisation, la rampe comprend une section de collecte configurée pour collecter au moins une partie de l'eau de la chasse d'eau.

Dans un mode de réalisation, la section de collecte est configurée pour être à proximité, avantageusement en contact, de la paroi interne de la cuvette des toilettes.

Dans un mode de réalisation, la rampe est arrangée entre la portion d'extrémité proximale et la portion d'extrémité distale.

Dans un mode de réalisation, la rampe fait partie de la portion intermédiaire.

Dans un mode de réalisation, la rampe présente une forme de rigole.

Dans un mode de réalisation, la rampe présente une largeur supérieure à 1cm

Dans un mode de réalisation, le bras de fixation comprend en outre une portion d'extrémité distale configurée pour coopérer avec la cuvette de toilettes et une portion intermédiaire arrangée entre la portion d'extrémité proximale et la portion d'extrémité distale, la portion intermédiaire comprenant une portion de liaison et la rampe.

Dans un mode de réalisation, la portion de liaison comprend une zone de passage permettant le passage de l'eau du flux d'eau.

Dans un mode de réalisation, la zone de passage est connectée à la section de collecte.

Dans un mode de réalisation, la section de collecte comprend une ailette.

Dans un mode de réalisation, la rampe est arrangée entre la portion d'extrémité proximale et la portion d'extrémité distale.

Dans un mode de réalisation, la zone de passage est définie par une ouverture dans la portion de liaison.

Dans un mode de réalisation, la zone de passage est définie par un rétrécissement de section au niveau de la portion de liaison.

Dans un mode de réalisation, la rampe est arrangée entre la portion d'extrémité proximale et la portion de liaison.

Dans un mode de réalisation, la portion de liaison comprend une zone de passage reliée à la section de collecte de la rampe.

Dans un mode de réalisation, la rampe et la portion de liaison formant un U ou un V.

Dans un mode de réalisation, la section de collecte est déportée en direction de la cuvette.

Dans un mode de réalisation, la portion de liaison est arrangée entre la section de transvasement et la portion d'extrémité distale.

Dans un mode de réalisation, la section de collecte de la rampe s'étend à partir d'une jonction entre la portion de liaison et la section de transvasement, la section de collecte comprenant une extrémité libre du côté opposé à la jonction.

Dans un mode de réalisation, la section de collecte comprend une ailette.

Dans un mode de réalisation, la portion de liaison comprend une zone de passage, à partir de laquelle s'étend la section de collecte de la rampe, en direction opposé à la section de transvasement.

Dans un mode de réalisation, la section de collecte de la rampe fait saillie depuis la zone de passage.

Dans un mode de réalisation, la section de collecte comprend une ou plusieurs ailettes qui s'étendent latéralement depuis la jonction.

Dans un mode de réalisation, la section de collecte comprend deux ailettes s'étendant de part et d'autre de la jonction.

Dans un mode de réalisation, la section de collecte comprend un entonnoir qui s'étend autour de la jonction.

Dans un mode de réalisation, le dispositif d'analyse d'urine comprend un orifice de collecte, l'orifice de collecte étant configuré pour collecter le liquide de l'extérieur du boîtier vers l'intérieur du boîtier.

Dans un mode de réalisation, l'orifice de collecte est situé sur une face arrière, opposée à la face avant.

Dans un mode de réalisation, la face arrière comprend au moins un écarteur faisant saillie de la face arrière et configuré pour être en contact avec la cuvette des toilettes.

L'invention concerne également un bras de fixation configuré pour positionner un dispositif d'analyse d'urine entièrement dans une cuvette de toilettes, le dispositif d'analyse d'urine comprenant un boîtier comprenant une face avant destinée à recevoir un flux d'urine provenant directement d'un utilisateur urinant dans la cuvette des toilettes, le bras de fixation comprenant une rampe d'amenée d'eau configurée pour orienter un flux d'eau provenant d'une chasse d'eau de la cuvette de toilettes vers la face avant du boîtier.

### Présentation des figures

D'autres caractéristiques, détails et avantages apparaîtront à la lecture de la description détaillée ci-après, et à l'analyse des dessins annexés, sur lesquels :
- [FIG. 1] : la figure 1 présente une représentation schématique et simplifiée d'un dispositif d'analyse d'urine installé dans une cuvette de toilettes,
- [FIG. 2] : la figure 2 présente une vue éclatée du dispositif d'analyse d'urine, dans laquelle la station et la cartouche sont visibles,
- [FIG. 3] : la figure 3 présente une vue détaillée d'une cartouche selon un mode de réalisation,
- [FIG. 4] : la figure 4 présente une vue en coupe d'une cartouche et d'une station selon un mode de réalisation, à l'emplacement d'un analyseur optique de la station,
- [FIG. 5] : la figure 5 présente une vue latérale du dispositif d'analyse d'urine installé dans une cuvette de toilettes avec un bras de fixation dans une configuration de repos,
- [FIG. 6] : la figure 6 présente une vue latérale d'un ensemble d'analyse selon la figure 5 mais avec le bras dans une configuration déployée,
- [FIG. 7] : la figure 7 présente une vue en perspective (a) et une vue latérale (b) d'un bras de fixation de l'ensemble des figures 5 et 6,
- [FIG. 8] : la figure 8 présente une vue latérale partielle (a) d'un dispositif d'analyse d'urine et une vue de face (b) du bras de fixation tel qu'illustré en figures 5 à 7,
- [FIG. 9] : la figure 9 présente une vue en perspective d'une variante du bras de fixation de l'ensemble de la figure 5,
- [FIG. 10] : la figure 10 présente une vue latérale partielle (a) d'un dispositif d'analyse d'urine et une vue de face (b) d'une variante du bras de fixation de l'ensemble de la figure 5,
- [FIG. 11] : la figure 11 présente une vue latérale partielle (a) d'un dispositif d'analyse d'urine et une vue de face (b) d'une variante du bras de fixation de l'ensemble de la figure 5,
- [FIG. 12] : la figure 12 présente une vue latérale partielle (a) d'un dispositif d'analyse d'urine et une vue de face (b) selon une variante d'un autre mode de réalisation du bras de fixation de l'ensemble de la figure 5,
- [FIG. 13] : la figure 13 présente une vue latérale partielle (a) d'un dispositif d'analyse d'urine et une vue de face (b) selon une autre variante de l'autre mode de réalisation du bras de fixation de l'ensemble de la figure 5,
- [FIG. 14] : la figure 14 présente une vue latérale partielle (a) d'un dispositif d'analyse d'urine et une vue de face (b) selon une autre variante de l'autre mode de réalisation du bras de fixation de l'ensemble de la figure 5,
- [FIG. 15] : la figure 15 présente une vue latérale partielle (a) d'un dispositif d'analyse d'urine et une vue de face (b) selon une autre mode de réalisation du bras de fixation de l'ensemble de la figure 5,
- [FIG. 16] : la figure 16 présente une vue latérale partielle (a) d'un dispositif d'analyse d'urine et une vue de face (b) d'une variante de l'autre mode de réalisation du bras de fixation de l'ensemble de la figure 15
- [FIG. 17] : la figure 17 présente une vue latérale partielle (a) d'un dispositif d'analyse d'urine et une vue de face (b) d'un autre mode de réalisation du bras de fixation de l'ensemble de la figure 5.

### Description détaillée

La présente description présente différents exemples de dispositif d'analyse d'urine comprenant une station et une cartouche telle que divulguée dans les documents WO2021/175909 et WO2021/175944, ci-après dénommés WO'909 et WO'944. Des variantes des stations sont présentées dans les documents WO2023036805, WO2023036806, WO2023036808, WO2023036809, ci-après dénommés WO'80X.

Les paragraphes suivants expliquent le principe général d'un dispositif d'analyse d'urine, mais tous les détails des documents WO'909 et WO'933 (ainsi que de tous les documents PCT susmentionnés) sont applicables.

### Orientation

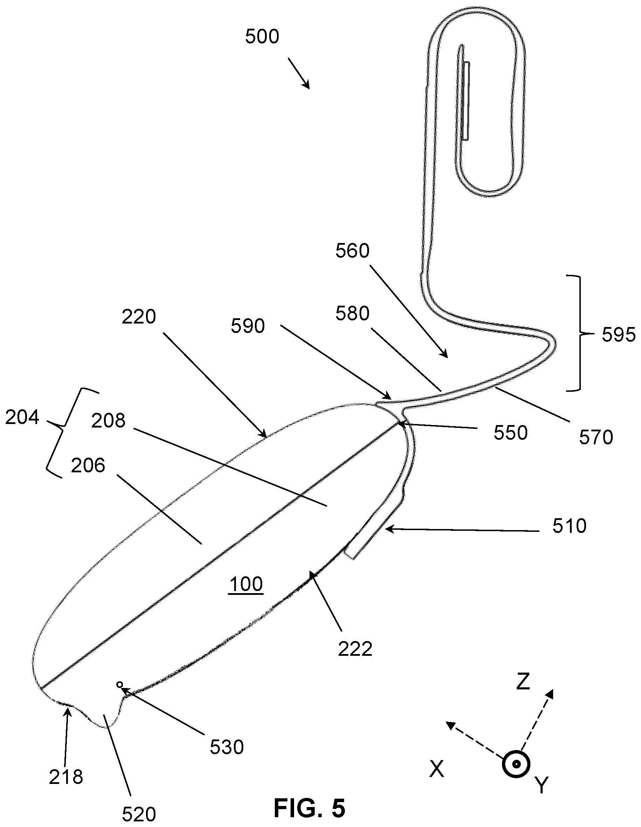

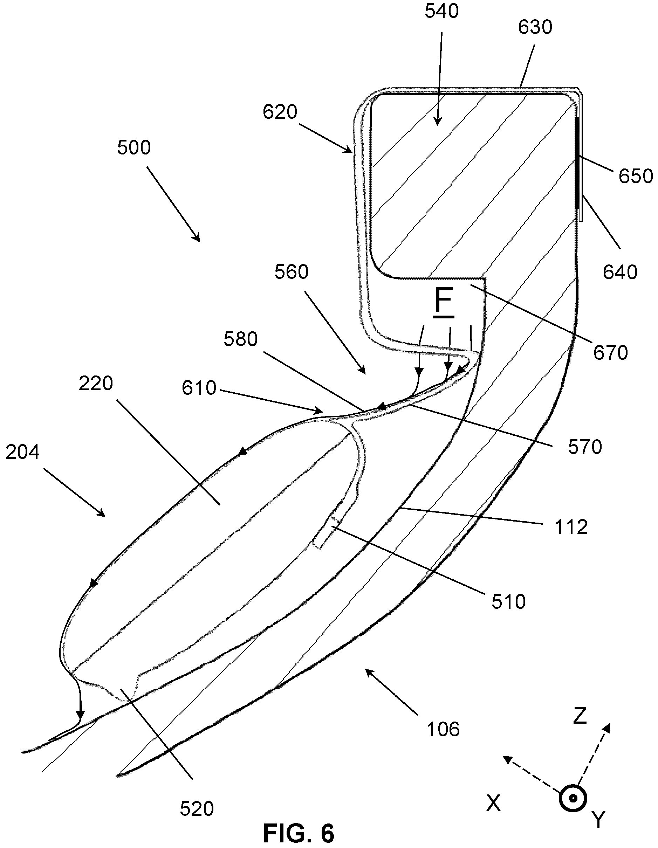

Dans la suite de l'exposé, les notions de "haut" et "bas", "supérieur" et "inférieur", etc. sont définies par rapport à une direction Z, telle que définie sur les figures 5 et 6. Le haut selon la direction Z est défini dans une position d'utilisation normale du dispositif d'analyse d'urine fixé dans la cuvette des toilettes.

### Forme générale du boîtier

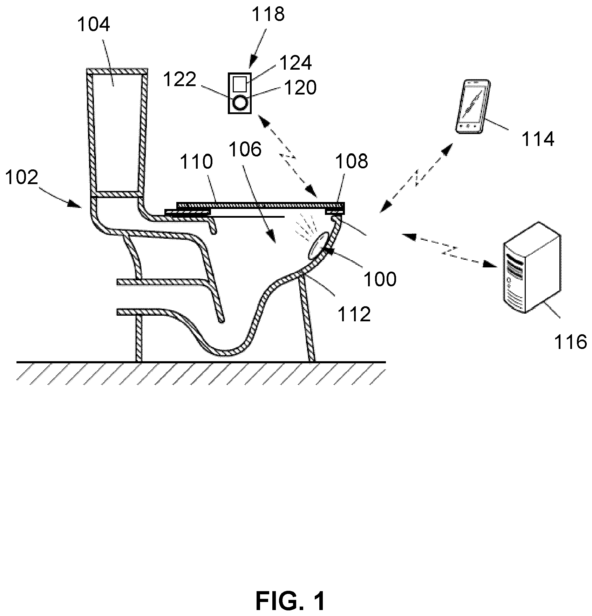

La figure 1 illustre schématiquement un dispositif d'analyse 100 (appelé "dispositif 100") pour l'analyse d'urine installé dans les toilettes 102. Les toilettes 102 comprennent généralement un réservoir d'eau 104, une cuvette 106, un siège 108 et un couvercle de siège 110. Le dispositif d'analyse 100 est configuré pour être placé entièrement dans la cuvette des toilettes. Par "dans la cuvette", on entend "placé dans le volume intérieur défini par la cuvette". Le dispositif d'analyse 100 est disposé de manière amovible dans les toilettes 102. Par exemple, le dispositif d'analyse 100 peut être facilement retiré des toilettes pour remplacer une cartouche, puis replacé dans les toilettes 102. Le dispositif d'analyse 100 est placé sur une paroi interne 112 de la cuvette 106 des toilettes. Le dispositif d'analyse 100 est placé de telle sorte qu'il se trouve généralement sous le jet d'urine d'un utilisateur, de sorte que lorsqu'un utilisateur urine (généralement en position assise), l'urine entre en contact avec le dispositif d'analyse 100. Le dispositif d'analyse 100 peut communiquer à distance avec une entité distante, telle que le smartphone 114 ou un serveur 116.

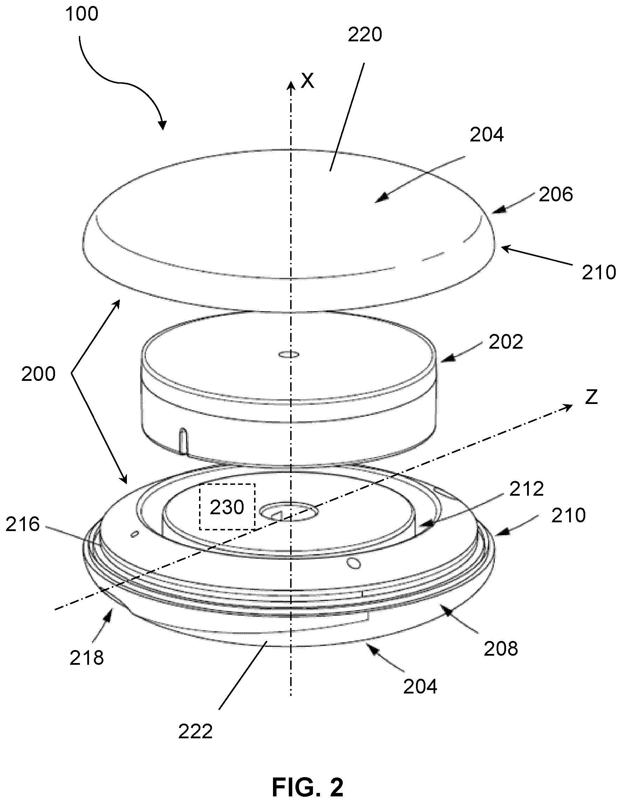

Comme l'illustre plus en détail la figure 2, le dispositif d'analyse 100 peut comprendre une station 200 et une cartouche 202, montée de manière amovible sur la station 200. La station 200 peut comprendre un boîtier 204 qui peut comporter deux coques, notamment une coque avant 206 et une coque arrière 208. La coque avant 206 et la coque arrière 208 peuvent coopérer l'une avec l'autre par l'intermédiaire d'un mécanisme de fixation 216, dans un plan normal à l'axe X. La coque avant 206 et la coque arrière 208 peuvent être assemblées de manière réversible, par exemple par vissage ou par clipsage. Dans une variante, la coque avant 206 et la coque arrière 208 peuvent être assemblées de manière permanente, par exemple par collage, clipsage, magnétisation, ou soudage par ultrasons. Bien entendu, d'autres moyens de fixation peuvent être utilisés pour assembler les deux coques.

En particulier, comme illustré sur la figure 2, la coque avant 206 et la coque arrière 208 sont vissées l'une à l'autre. Ensuite, une partie interne de la coque avant 206 comprend un filetage. Le filetage de la coque avant 206 est destiné à coopérer avec un filetage complémentaire de la coque arrière 208. Cela permet de démonter facilement le boîtier 204 pour accéder à l'assemblage de test à l'intérieur du boîtier.

Un joint peut être présent entre la coque avant 206 et la coque arrière 208. Ainsi, le boîtier 204 est étanche. Seuls les orifices de collecte et de drainage relient l'extérieur et l'intérieur de la boîtier 204, comme décrit plus en détail ci-dessous.

Comme on peut le voir sur les figures, le boîtier 204 peut avoir une forme globale extérieure de galet circulaire. En d'autres termes, le boîtier 204 présente une forme sphéroïde. L'axe X est la ligne centrale du boîtier. Avantageusement, la coque avant 206 peut être sensiblement symétrique en rotation, ce qui donne un aspect aérodynamique au dispositif une fois installé. Le boîtier 204 sert de collecteur d'urine.

Le boîtier 204 comprend une face avant 220 pour recevoir un flux d'urine directement d'un utilisateur urinant sur les toilettes et une face arrière 222 opposée à la face avant 220. Comme illustré à la figure 2, la face avant 220 peut être disposée sur la coque avant 206 et la face arrière 222 peut être disposée sur la coque arrière 208. La face avant 220 est orientée vers l'intérieur de la cuvette 106. La face avant 220 est donc destinée à recevoir l'urine lorsque l'utilisateur urine en s'asseyant sur les toilettes 102. Comme le montrent les figures 5 et 6, la face arrière 222 fait face à la paroi intérieure 112 de la cuvette 106. Par la suite de la description, il est entendu par un objet faisant face à la paroi de la cuvette, un objet faisant face à la paroi de la cuvette la plus proche de l'objet en question, et non la paroi de la cuvette en vis-à-vis de l'autre côté du volume interne de la cuvette.

La face avant 220 et la face arrière 222 comportent chacune un bord incurvé 210. Les bords incurvés 210 respectifs de la face avant et de la face arrière se rejoignent au niveau d'une zone de jonction équatoriale. Ainsi, la surface extérieure du boîtier 204, constituée de la face avant 220 et de la face arrière 222, est définie par des lignes courbes et forme un objet généralement convexe.

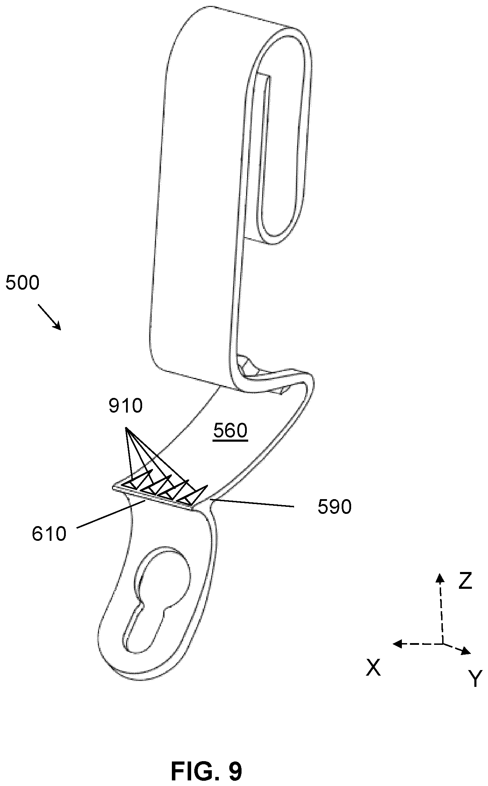

En référence aux figures 5 et 6, le boîtier 204 présente un sommet 550. Le sommet 550 est l'endroit le plus haut du boîtier 204 selon l'axe Z dans une position d'utilisation normale du boîtier 204 lorsqu'il est placé dans la cuvette 106 des toilettes. Le sommet 550 peut se trouver à la jonction entre la face avant 220 et de la face arrière 222. Dans un mode de réalisation où le boîtier 204 est de forme parallélépipédique comme illustré sur la figure 9, le sommet 550 est situé sur la face supérieure, au niveau de la jonction entre la face avant 220 et de la face arrière 222.

La surface extérieure de la face avant 220 peut être lisse. En d'autres termes, la face avant 220 est dépourvue de crêtes ou de rainures. Ainsi, le flux d'urine entrant en contact avec la face avant 220 s'accroche et s'étale sur la face avant 220. La face avant 220 peut être sensiblement symétrique en rotation autour de l'axe X.

La surface extérieure du boîtier 204 peut également être blanche ou de couleur claire. La couleur de la surface extérieure peut être similaire à celle des toilettes, ce qui accroît la discrétion du dispositif.

Le boîtier 204 peut avoir un diamètre, mesuré dans la direction orthogonale à l'axe X, compris entre 50 mm et 150 mm. Le boîtier 204 peut avoir une épaisseur, mesurée dans la direction de l'axe X, comprise entre 15 mm et 50 mm. Ainsi, le boîtier 204 est suffisamment compact pour être entièrement logé dans la cuvette des toilettes. Le dispositif d'analyse d'urine 100 est discret. De plus, le boîtier 204 est suffisamment grand pour entrer systématiquement en contact avec l'urine reçue dans la cuvette. L'utilisateur peut alors uriner dans les toilettes sans se soucier du dispositif d'analyse d'urine, ou alternativement viser sommairement.

Selon un autre aspect, dans un mode de réalisation, le boîtier 204 présente un facteur de forme général tel que le rapport entre l'épaisseur et le diamètre est compris entre 0,2 et 0,5, et même de préférence entre 0,3 et 0,4. De telles proportions rappellent un galet naturel et donnent au dispositif un aspect apaisant.

De préférence, le boîtier 204 est constitué d'un matériau hydrophile. Par exemple, le matériau du boîtier 204 peut être : une céramique, un polyamide (PA), un silicone ou un polymère hydrophile. La surface extérieure du boîtier 204 peut également être traitée avec un traitement de surface hydrophile, par exemple acuWet^{®} d'Aculon, un polymère hydrophile, ou Pebax^{®} d'Arkema.

### Ensemble de test

Un ensemble de test 230 est disposé à l'intérieur du boîtier 204 et configuré pour effectuer une analyse sur l'urine collectée par l'orifice de collecte. La station 200 comprend un compartiment annulaire 212, situé à l'intérieur du boîtier 204, disposé autour d'un axe de rotation X. Le compartiment annulaire 212 est configuré pour recevoir au moins partiellement la cartouche 202 montée de manière rotative autour de l'axe de rotation X (une fois en position dans le compartiment annulaire 212). La cartouche 202 comprend une pluralité de supports de test qui comprennent chacun au moins un réactif urinaire, par exemple un réactif sec, la pluralité de supports de test étant disposée le long d'un cercle ou d'un arc de cercle autour de l'axe de rotation X. Dans un mode de réalisation, les supports de test sont des bandelettes de test. Les supports de test peuvent être enfermés, par exemple individuellement, dans une chambre.

Le compartiment annulaire 212 s'étend typiquement sur 360° et forme une gorge configurée pour recevoir au moins partiellement la cartouche 202.

La station 200 comprend un orifice de collecte 218, située par exemple sur la coque arrière 208. Comme cela sera expliqué plus en détail ci-dessous, l'orifice de collecte 218 est configurée pour collecter l'urine s'écoulant sur la surface du boîtier 204. La station 200 comprend également un orifice de drainage 530, visible sur la figure 6, configurée pour drainer le liquide hors du dispositif 100.

L'ensemble de test 230 peut comprendre une pompe, un injecteur et un analyseur. La pompe aspire l'urine de l'orifice de collecte 218, puis l'injecteur injecte l'urine sur un ou plusieurs supports de test de la cartouche et l'analyseur obtient certaines valeurs de propriétés (par exemple, des propriétés physiques/chimiques, telles que la couleur) des supports de test après qu'ils sont entrés en contact avec l'urine. Dans un cas, l'analyseur est un analyseur optique configuré pour analyser les propriétés optiques du support de test. L'injecteur et la cartouche peuvent se déplacer l'un par rapport à l'autre afin que l'injecteur puisse ouvrir (par exemple, percer) la chambre, par exemple à l'aide d'une aiguille ou d'un dispositif semblable à une aiguille.

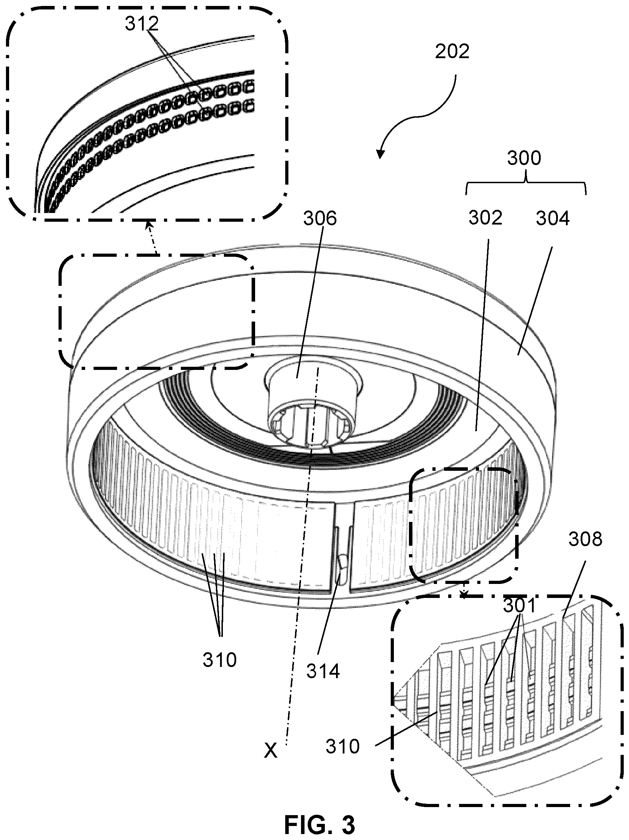

La figure 3 montre une vue éclatée de la cartouche 202. La cartouche 202 comprend au moins un support de test 301, notamment plusieurs supports de test 301 configurés pour recevoir l'urine de l'injecteur. Chaque support de test 301 contient un réactif urinaire qui réagit de manière spécifique au contact de l'urine. La cartouche 202 comprend un support rotatif 300, configuré pour être entraîné en rotation par la station 200. Dans le cadre d'une utilisation normale de la cartouche 202 et du dispositif 100, les supports de test 301 restent fixés au support rotatif et ne se déplacent pas par rapport à ce dernier.

Dans une réalisation, le support rotatif 300 a une forme de cylindre circulaire droit d'au moins 80% d'une forme de cylindre creux s'étendant annulairement autour d'un axe qui est, lorsque la cartouche 202 est montée dans la station 200, l'axe de rotation X. Chaque support de test 301 peut être une bandelette de test. Le support rotatif 300 peut comprendre une partie annulaire 302 et une partie cylindrique 304, qui s'étend à partir d'une extrémité radiale extérieure de la partie annulaire 302. La partie cylindrique 304, lorsqu'elle est utilisée, est logée à l'intérieur du compartiment annulaire 212. Les supports de test 301 sont positionnés le long de la portion cylindrique 304, de manière à pouvoir défiler sélectivement et/ou successivement devant l'injecteur et l'analyseur. Par exemple, les supports de test 301 font partie d'un support 308, qui comprend plusieurs chambres 310, séparées les unes des autres le long d'un périmètre autour de l'axe X. Au moins une bandelette de test est reçue dans une chambre 310.

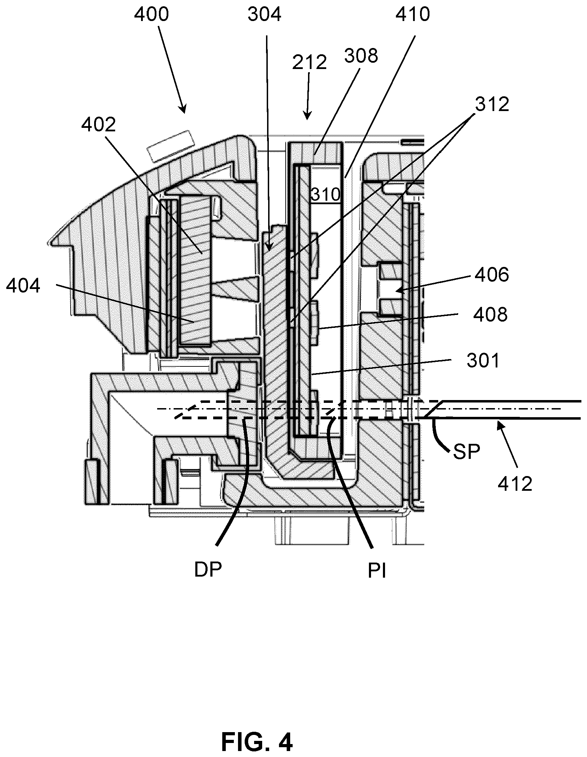

Les chambres 310 sont disposées les unes à côté des autres en forme de cylindre circulaire droit d'au moins 80 % du cercle. Pour permettre à la lumière de passer, le support 308 comprend au moins une ouverture 312 par chambre 310 (représentée dans le zoom supérieur gauche où le support rotatif est représenté comme transparent). Les chambres 310 sont toutes à égale distance de l'axe de rotation X, de sorte que l'injecteur peut injecter sélectivement l'urine une fois que la chambre souhaitée est positionnée à l'endroit voulu face à l'injecteur. L'injecteur peut se déplacer vers la chambre 310 et percer un couvercle fermant la chambre 310 (visible sur la figure 4). Un orifice de vidange 314 est prévue dans le support rotatif 300 pour permettre l'évacuation de l'urine de l'injecteur vers l'extérieur du dispositif 100, via l'orifice de drainage 530 disposée sur le boîtier 204.

La partie annulaire 302 du support rotatif 300 reste à l'extérieur du compartiment annulaire 212 pour renforcer la partie cylindrique et/ou entraîner en rotation la cartouche 202. À cette fin, la partie annulaire 302 peut comprendre un accouplement mécanique 306, qui coopère avec un arbre de la station 200.

Les dimensions relatives à la cartouche 202 sont divulguées dans les documents WO'909, WO'933 et WO'80X. La dimension maximale du dispositif 100 transversalement à l'axe de rotation X est inférieure à 15 cm, voire inférieure à 10 cm. La dimension maximale du dispositif le long de l'axe de rotation X est inférieure à 5 cm.

La figure 4 montre plus en détail l'interaction entre la cartouche 202 et la station 200 lorsque ou après l'activation de l'injecteur. L'analyseur 400 comprend au moins une source lumineuse 402, 404 (par exemple, deux sources lumineuses) et au moins un capteur optique 406. La lumière va de la source lumineuse 402, 404 au capteur optique 406 en traversant la cartouche 202 et en particulier la partie cylindrique 304, l'ouverture 312 du support 308, le support de test 301 et donc le réactif 408.

Dans un mode de réalisation, l'analyseur 400 est configuré pour mesurer l'absorbance d'une partie des supports de test 301 (notamment la ligne de test et/ou la ligne de contrôle d'une bandelette comme cela sera expliqué plus loin). L'absorbance est détectée par la source lumineuse (par exemple une LED) qui peut faire passer de la lumière à travers la bande, et le capteur optique qui reçoit le spectre avec une dizaine de longueurs d'onde.

Dans une variante, le capteur de lumière est une caméra capable de détecter un changement de couleur, notamment un changement d'intensité de couleur, d'une partie des supports de test 301 (notamment la ligne de test et/ou la ligne de contrôle d'une bandelette comme cela sera expliqué ci-dessous). La caméra peut détecter une couleur en valeurs RVB par exemple.

L'injecteur comprend une extrémité d'injection 412 (par exemple une aiguille), qui peut être déplacée entre plusieurs positions, représentées par des lignes en tirets sur la figure 4. Dans une position d'attente SP, l'extrémité d'injection 412 est à l'extérieur de la cartouche 202 (dans une position la plus intérieure), de sorte que la cartouche 202 peut tourner librement dans le compartiment annulaire 212 ; dans une position d'injection IP, l'extrémité d'injection 412 a percé le couvercle 410 pour accéder à l'intérieur de la chambre 310 et peut injecter un peu d'urine sur le support de test 301 ; dans la position de vidange DP, l'extrémité d'injection 412 est capable d'évacuer l'urine par l'orifice de vidange 314 du support rotatif 300.

En position SP, l'injecteur est situé radialement à l'intérieur de la chambre annulaire. Ceci permet de maximiser le rayon du compartiment annulaire tout en minimisant la taille de la station 200.

Dans une variante de l'ensemble de test 230 divulgué ci-dessus, l'homme de métier comprendra que chaque ensemble de test configuré pour analyser l'échantillon d'urine collecté par l'orifice de collecte 218 peut être disposé à l'intérieur du boîtier 204.

### Orifice de collecte

L'orifice de collecte 218 est configuré pour recevoir l'urine qui s'écoule par gravité sur la surface extérieure du boîtier 204. L'urine est collectée directement sur la face avant 220 et la face arrière 222 du boîtier 204.

L'orifice de collecte 218 est une ouverture configurée pour collecter le liquide, de sorte que le liquide puisse pénétrer dans le dispositif d'analyse d'urine. L'orifice de collecte 218 est généralement circulaire, avec un diamètre de préférence compris entre 0,3 mm et 2 mm. Le diamètre de l'orifice de collecte peut être choisi pour maximiser le volume d'urine collecté sur la surface extérieure du boîtier 204.

Comme on peut le voir sur les figures, l'orifice de collecte 218 est situé sur la face arrière 222. Ainsi, l'orifice de collecte 218 fait face à la paroi interne 112 des toilettes lorsque le dispositif d'analyse d'urine 100 est positionné dans les toilettes. Cette position permet à l'orifice de collecte 218 d'être cachée à la vue de l'utilisateur par la face avant 220 du boîtier. La face avant 220 visible par l'utilisateur ressemble à un simple galet uniforme, comme déjà mentionné, sans points singuliers ni trous. Il convient également de noter que cette position empêche l'introduction de contaminants ou d'éléments qui pourraient obstruer l'ensemble de test 230.

L'orifice de collecte 218 est situé sur une partie inférieure de la face arrière 222. Par "sur une partie inférieure de la face arrière", on entend "sur le dernier quart de la face selon la direction Z à partir de l'extrémité inférieure du boîtier 204". L'extrémité inférieure fait face au fond de la cuvette 106 lorsque le boîtier 204 est positionné dans la cuvette. L'extrémité inférieure se situe à l'opposé du sommet 550. Cette position correspond à une position normale d'utilisation. Cette position permet à l'urine d'être collectée par gravité sur la majeure partie de la surface extérieure du boîtier 204.

En particulier, la distance séparant l'orifice de collecte 218 d'un bord inférieur du boîtier 204 est inférieure à 40 mm, de préférence inférieure à 20 mm. Comme illustré, selon un mode de réalisation particulier, l'orifice de collecte 218 est disposée à quelques millimètres au-dessus du bord inférieur du boîtier 204. En variante, l'orifice de collecte 218 peut se trouver sur le bord inférieur (le bord inférieur étant défini lorsque le dispositif 100 est placé pour être utilisé dans les toilettes).

L'orifice de collecte 218 peut être couvert par un filtre à mailles. Le filtre à mailles est par exemple de forme oblongue et recouvre l'orifice de collecte 218. L'ouverture moyenne des mailles du filtre est, par exemple, de 20 microns. Le filtre à maille empêche l'introduction de contaminants ou d'éléments susceptibles d'obstruer l'ensemble de test 230 et filtre l'urine reçue dans l'orifice de collecte 218. La maille du filtre peut être en métal.

### Ecarteur

En référence aux figures 5 et 6, dans un mode de réalisation, le dispositif 100 peut comprendre au moins un écarteur 520 faisant saillie de la face arrière 222 et configuré pour être en contact avec la cuvette 106, en particulier en contact avec la paroi interne 112 de la cuvette 106. L'écarteur 520 est disposé sur la face arrière 222. Dans un mode de réalisation, l'écarteur 520 est disposé sur la face arrière 222 de sorte que l'orifice de collecte 218 se trouve à une certaine distance de la cuvette 106 lors d'une utilisation normale du dispositif. Cela évite que la paroi interne 112 de la cuvette 106 n'obstrue l'entrée de l'orifice de collecte 218 et/ou ne constitue un goulot d'étranglement pour l'écoulement d'un mélange d'urine, d'eau et d'impuretés provenant de la cuvette (qui pourrait être collecté par l'orifice de collecte dans d'autres circonstances).

Grâce à l'au moins un écarteur 520, l'urine s'écoule sur le boîtier 204 (en particulier sur la face arrière 222) vers l'orifice de collecte 218 sans être gênée par la cuvette 106 comme cela pourrait se produire avec un dispositif tel que décrit dans les documents WO'909 et WO'944.

Dans un mode de réalisation, les écarteurs 520 sont situés sur la partie inférieure du dispositif d'analyse d'urine 100, et plus particulièrement sur la partie inférieure de la face arrière 222, à côté de l'orifice de collecte 218.

L'écarteur 520 comprend typiquement une arête. Lors de l'utilisation, une partie de l'arête est en contact avec la cuvette des toilettes.

En particulier, le dispositif 100 peut comprendre deux écarteurs 520, avantageusement disposés de chaque côté de l'orifice de collecte 218, le long de la direction transversale Y. Les deux écarteurs 520 peuvent être disposés de chaque côté de l'orifice de collecte 218, le long de l'orifice de collecte 218. Les deux écarteurs 520 peuvent être symétriques par exemple le long d'un plan vertical XZ, dans une position d'utilisation normale du dispositif 100 ou par exemple autour de l'orifice de collecte 218.

L'écarteur 520 a été décrit en détail dans la demande de brevet EP23192265.

### FIXATION

Le boîtier 204 est destiné à être placé sur la paroi interne 112 de la cuvette 106. Le boîtier 204 est fixé par un bras de fixation 500. Le bras de fixation 500 est configuré pour fixer le dispositif d'analyse d'urine 100 dans la cuvette 106 des toilettes. Le bras de fixation 500 comprend plusieurs sections qui ont des fonctions différentes et qui seront décrites en détail par la suite. Toutefois, le bras de fixation 500 est typiquement réalisé en un seul matériau, par exemple du plastique, par moulage.

Le dispositif d'analyse 100 comprend un élément d'attache 510 disposé sur la face arrière 222 du boîtier. L'élément d'attache 510 est configuré pour coopérer avec un bras de fixation 500.

L'élément d'attache 510 est disposé au niveau d'un plan médian XZ du boîtier 204. En d'autres termes, l'élément d'attache 510 est disposé au niveau d'un plan de symétrie vertical du boîtier 204.

Comme on peut le voir sur les figures, l'élément d'attache 510 peut être disposé sur la partie supérieure du boîtier 204. Par "sur la partie supérieure du boîtier", on entend "sur le premier quart le long de la direction Z à partir du sommet du boîtier 204". En particulier, l'élément d'attache 510 est disposé entre 15% et 25% de la face arrière 222 en partant du sommet 550 du boîtier 204.

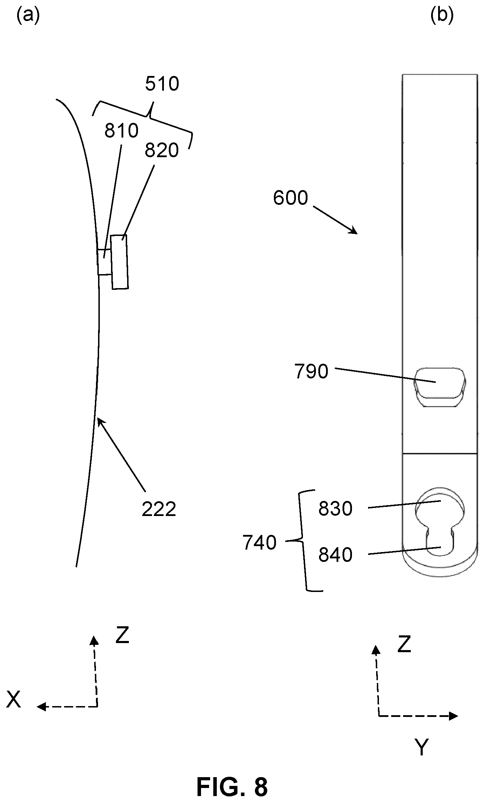

Comme visible sur la figure 8, l'élément d'attache 510 peut être un ergot. L'ergot fait saillie hors de la face arrière 222 du boîtier 204. Selon un mode de réalisation, l'ergot s'étend depuis la face arrière 222 selon un corps 810 puis une tête 820 d'étendue transversale supérieure au corps 810.

En variante non représentée, l'élément d'attache 510 est un aimant propre à coopérer avec un autre aimant placé sur le bras de fixation 500.

En variante non représentée, l'élément d'attache 510 comprend une partie adhésive propre à coopérer avec le bras de fixation 500.

### Bras de fixation

Selon un mode de réalisation, le bras de fixation 500 peut être amovible. Autrement dit, l'utilisateur peut séparer le bras de fixation 500 de l'élément d'attache 510 et donc du boitier. Le bras de fixation 500 peut être ainsi aisément remplacé et une autre forme de bras de fixation peut être par exemple utilisée.

Le bras de fixation 500 peut être flexible. Autrement dit, le bras de fixation 500 peut être déformé par l'utilisateur ou par la gravité du dispositif 100 placé dans les toilettes. Ceci permet ainsi une meilleure adaptation du bras de fixation aux différentes formes de toilettes et assure que le boîtier 204 touche la paroi interne 112 des toilettes. En particulier, le bras de fixation 500 est fait en un matériau plastique, par exemple du nylon ou un matériau métallique, par exemple de l'acier.

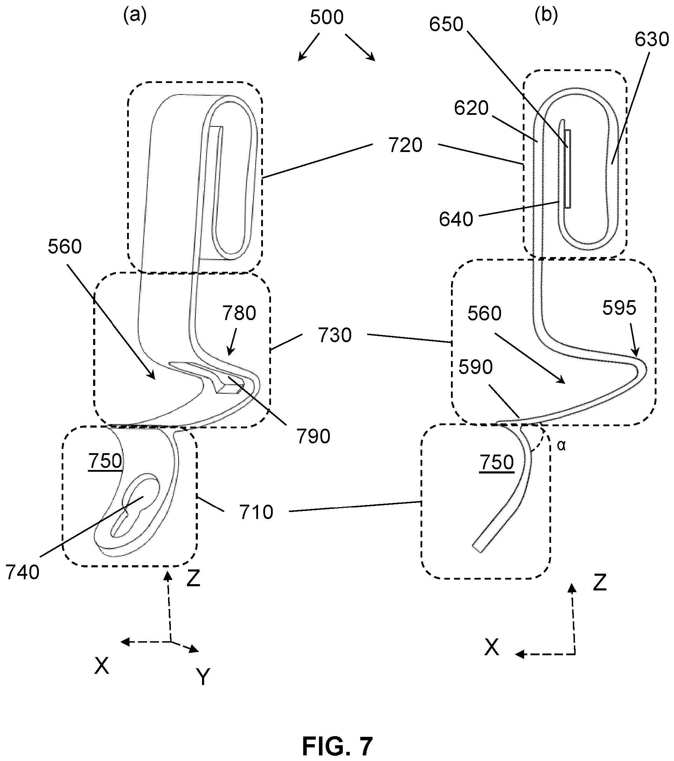

Comme visible sur la figure 7, le bras de fixation 500 peut présenter une forme aplatie dans une section transverse au plan médian XZ. Ceci permet une flexibilité du bras de fixation 500 dans le plan médian XZ tout en limitant la flexibilité selon la direction transversale Y.

Le bras de fixation 500 peut s'étendre selon une hauteur selon l'axe Z comprise entre 5 cm et 15 cm, dans une configuration de repos (c'est-à-dire ni étiré ni comprimé). Le bras de fixation 500 présente par exemple une largeur selon l'axe Y comprise entre 1 cm et 2 cm. Le bras de fixation 500 présente par exemple une épaisseur comprise entre 1 mm et 2 mm.

### Portion d'extrémité proximale

En référence à la figure 7, le bras de fixation 500 comprend une portion d'extrémité proximale 710 configurée pour coopérer avec l'élément d'attache 510 du dispositif d'analyse d'urine 100. Par la suite, les termes "proximal" et "distal" sont définis selon la proximité au dispositif d'analyse d'urine 100.

Lorsque l'élément d'attache 510 est un ergot, la portion d'extrémité proximale 710 comprend une cavité 740 destinée à recevoir l'élément d'attache 510. La cavité 740 peut être traversante.

En référence à la figure 8, la cavité 740 présente une première section 830 de largeur transversale maximale supérieure à la largeur de la tête 820 de l'ergot et une deuxième section 840 de largeur transversale maximale inférieure à la largeur de la tête 820 de l'ergot. La deuxième section 840 est également de largeur transversale supérieur au corps 810 de l'ergot. Ainsi, lorsque l'utilisateur veut assembler le bras de fixation 500 avec le dispositif 100, il/elle introduit l'ergot dans la première section 830 puis fait glisser l'ergot dans la deuxième section 840. La tête 820 de l'ergot étant plus large que la deuxième section 840, le bras de fixation 500 est maintenu solidaire du dispositif 100. A l'inverse, lorsque l'utilisateur veut désolidariser le bras de fixation 500 du dispositif 100, il/elle glisse l'ergot de la deuxième section 840 vers la première section 830 afin de permettre le passage de la tête 820 de l'ergot à travers la cavité 740.

### Espace de réception

Comme visible sur les figures 6 à 10 et 12 à 17, la portion d'extrémité proximale 710 peut former un espace de réception 750 pour le boîtier 204. Lorsque le bras de fixation 500 coopère avec le boîtier 204, l'espace de réception 750 s'étend depuis l'élément d'attache 510 jusqu'à au moins un sommet 550 du boîtier 204. L'espace de réception 750 s'étend ainsi depuis la face arrière 222 jusqu'au moins le sommet 550. Dans un mode de réalisation, l'espace de réception 750 s'étend jusqu'à la face avant 220 du boîtier 204, comme illustré sur la figure 6.

L'espace de réception 750 est configuré pour recevoir au moins partiellement la partie supérieure de la face arrière 222 du boîtier 204, jusqu'au sommet 550. Dans un mode de réalisation, l'espace de réception 750 est de forme complémentaire à la partie supérieure de la face arrière 222 du boîtier 204, avec par exemple un jeu fonctionnel lorsque le dispositif 100 est installé dans la cuvette 106 des toilettes.

Dans un mode de réalisation, comme illustré sur les figures 6 à 8, l'espace de réception 750 est de forme arrondie, notamment de forme complémentaire avec la forme en galet du boîtier 204.

Dans un mode de réalisation, la portion d'extrémité proximale 710 comprend une butée 610. La butée 610 peut définir une extrémité de l'espace de réception 750 opposée à l'extrémité de l'espace de réception coopérant avec l'élément d'attache 510. La butée 610 est configurée pour bloquer un mouvement du dispositif d'analyse d'urine 100 autorisé par le bras de fixation 500, notamment autorisée par la liaison avec l'élément d'attache 510.

### Portion d'extrémité distale

Le bras de fixation 500 comprend en outre une portion d'extrémité distale 720. La portion d'extrémité distale 720 est opposée à la portion d'extrémité proximale 710. Comme visible sur la figure 6, la portion d'extrémité distale 720 est configurée pour coopérer avec la cuvette 106 de toilettes.

Dans un mode de réalisation illustré sur les figures, notamment les figures 5 à 7, la portion d'extrémité distale 720 comprend un crochet. En particulier, la portion d'extrémité distale 720 est déformable entre une configuration de repos, comme illustré sur les figures 5 et 7 et une configuration déployée, comme illustré en figure 6. En configuration de repos, la portion d'extrémité distale 720 est repliée sur elle-même ; en configuration déployée, la portion d'extrémité distale 720 est configurée pour être accrochée au rebord 540 de la cuvette 106 de toilettes. La portion d'extrémité distale 720 comprend trois parties allongées successives 620, 630, 640. Comme visible sur la figure 7, dans la configuration de repos, la troisième partie 640 est repliée entre la première partie 620 et la deuxième partie 630. Les trois parties 620, 630, 640 sont alors sensiblement parallèles entre elles. Comme visible sur la figure 6, dans la position déployée, la deuxième partie 630 et la troisième partie 640 sont à l'écart de la première partie 620, chaque paire de parties successives formant sensiblement un angle droit entre elles.

La troisième partie 640 peut comprend un bloc antidérapant 650, par exemple en gomme, permettant une meilleure adhérence du bras de fixation 500 avec la cuvette 106.

En variante non représentée, le bras de fixation 500 comprend un aimant propre à coopérer avec un autre aimant placé notamment sur l'élément d'attache 510.

En variante non représentée, le bras de fixation 500 comprend une partie adhésive propre à coopérer avec l'élément d'attache 510.

### Portion intermédiaire

Le bras de fixation 500 comprend en outre une portion intermédiaire 730 arrangée entre la portion d'extrémité proximale 710 et portion d'extrémité distale 720.

### Rampe

Le bras de fixation 500 comprend une rampe 560 d'amenée d'eau configurée pour orienter un flux d'eau F provenant d'une chasse d'eau 670 de la cuvette 106 de toilettes vers la face avant 220 du boîtier 204. Autrement dit, la rampe 560 forme un déflecteur orientant le flux F provenant d'une chasse d'eau 670 de la cuvette 106 de toilettes vers la face avant 220 du boîtier 204. La rampe 560 est arrangée entre la portion d'extrémité proximale 710 et la portion d'extrémité distale 720. Autrement dit, la rampe 560 fait partie de la portion intermédiaire 730.

La rampe 560 et la portion d'extrémité proximale 710 peuvent former entre elles une zone d'inflexion. Autrement dit, le bras de fixation 500 présente un changement de direction entre la rampe 560 et la portion d'extrémité proximale 710. En particulier, la rampe 560 et la portion d'extrémité proximale 710 forment entre elles une arête. La butée 610 est notamment formée par ce changement de direction. En particulier, comme illustré en figure 7 (b), la rampe 560 s'étend depuis le boîtier 204 en direction de la cuvette 106, et forme un angle α compris entre 0° et 90° avec un plan tangent à la face avant 220, notamment un angle α entre 30° et 60°.

La rampe 560 s'étend dans le prolongement du boîtier 204 et plus précisément dans le prolongement de la face avant 220 du boitier 204, ou au-dessus de celle-ci, de sorte que la transition entre la face avant 220 du boitier 204 et la rampe 560 soit continue ou quasiment continue (avec un jeu fonctionnel). Un tel arrangement permet un transfert d'eau de la rampe 560 vers la face avant 220 du boitier 204. En particulier, la rampe 560 comprend une surface arrière 570 faisant face à la paroi interne 112 de la cuvette 106 et une surface avant 580 opposée à la surface arrière 560. Tel que visible en figures 5 et 6, la surface avant 580 s'étend dans le prolongement de la face avant 220 du boîtier, notamment pour favoriser le passage de l'eau de la chasse sur la face avant 220 du boîtier. Alternativement (non illustré), la surface avant 580 peut être en surplomb du boîtier 204 (c'est-à-dire au-dessus du boîtier, formant ainsi un tremplin vers la face avant 220).

En particulier, la rampe 560 comprend une section de transvasement 590 configurée pour être à proximité du boîtier 204. La section de transvasement 590 peut être en contact avec le boîtier 204 ou espacé d'un léger jeu fonctionnel (un ou deux millimètre). La section de transvasement 590 est configurée pour favoriser le passage de l'eau du flux F de la chasse d'eau de la rampe 560 vers la face avant 220 du boiter 204. A cet effet, la section de transvasement 590 affleure la face avant 220 du boîtier 204. Autrement dit, la section de transvasement 590 et la face avant 220 forment une continuité pour le flux d'eau entre elles. Autrement dit, le plan tangent au boîtier 204 au niveau de la section de transvasement 590 est sensiblement parallèle au plan tangent à la surface avant 580 de la rampe 560 au niveau de la butée 610. Du fait de l'espace de réception 750, la section de transvasement 590 peut être en léger surplomb sur la face avant 220.

La rampe 560 comprend aussi une section de collecte 595 configurée pour collecter au moins une partie de l'eau de la chasse d'eau 670 afin de former le flux d'eau F. La section de collecte 595 est configurée pour être à proximité de la face interne 112 de la cuvette 106 des toilettes. La section de collecte 595 est déportée en direction de la paroi interne 112 de la cuvette 106 afin de capter l'eau de la chasse d'eau. Avantageusement, la section de collecte 595 est en contact avec la face interne 112 de la cuvette 106.

Ainsi, la section de collecte 595 a pour fonction de collecter l'eau de la chasse sur la rampe 560 et la section de transvasement 590 a pour fonction de transférer l'eau de la rampe 560 vers la face avant 220 du boitier 204.

Selon un mode de réalisation, la portion intermédiaire 730 peut comprendre une portion de liaison 780 et la rampe 560. En particulier, la portion de liaison 780 fait la liaison entre la rampe 560 et la portion d'extrémité distale 720.

La portion de liaison 780 comprend une zone de passage 790 de l'eau de la chasse d'eau F. La portion de liaison 780 est reliée à la section de collecte 595 de la rampe 560. De même, la zone de passage 790 est reliée à la section de collecte 595. La zone de passage 790 permet le passage de du flux d'eau F de la chasse d'eau depuis la section de collecte 595 vers la section de transvasement 590 de la rampe 560 (voir flux F en figure 6). En d'autres mots, la zone de passage 790 permet le passage du flux d'eau F de l'arrière du bras de fixation 500 vers l'avant du bras de fixation 500.

La rampe 560 peut être convexe longitudinalement, dans le plan médian XZ. La rampe 560 forme ainsi un tremplin pour le flux d'eau F de la chasse d'eau.

La rampe 560 peut être convexe transversalement, dans un plan transversal YZ. La rampe 560 forme ainsi une rigole pour le flux d'eau de la chasse d'eau 670.

La rampe 560 s'étend par exemple sur une longueur comprise entre 1 cm et 5 cm. La rampe 560 présente par exemple une largeur supérieure à 1 cm.

### Diffuseur

Dans un mode de réalisation illustré sur la figure 9, la rampe 560 peut comprendre un diffuseur 910 pour l'eau de la chasse. Le diffuseur 910 est configuré pour disperser l'eau de la rampe 560 sur la face avant 220 du boîtier 204. Le diffuseur 910 comprend par exemple des rainures, ou toute forme permettant de casser le flux F et de disperser l'eau de la chasse d'eau avant que le flux F s'écoule sur la face avant 220. Le diffuseur 910 permet ainsi d'augmenter la part de la face avant 220 qui est nettoyée par le flux F. Le diffuseur 910 peut notamment être positionné au niveau de la section de transvasement 590.

### Ailette

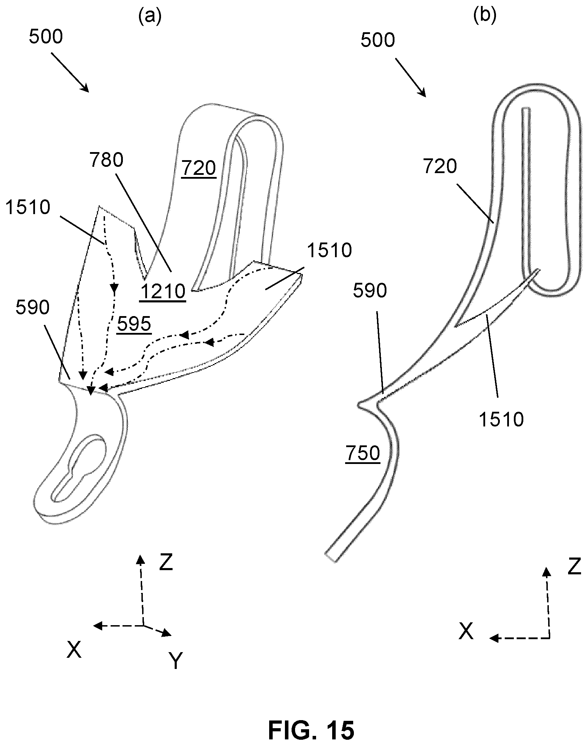

Dans un mode de réalisation, comme illustré sur la figure 15, la rampe 560 peut présenter au moins une ailette latérale, notamment deux ailettes latérales 1510. Chaque ailette latérale 1510 s'étend notamment transversalement depuis section intermédiaire 730. Chaque ailette latérale 1510 est configuré pour capter de l'eau de la chasse d'eau s'écoulant à côté du bras de fixation 500 et de le réorienter vers la section de transvasement 590 et ainsi vers le boîtier 204.

### Mode de réalisation rampe et portion de liaison "en série"

Les figures 5 à 11 présentent un mode de réalisation de l'ensemble d'analyse d'urine selon la présente description, dit "en série".

Comme visible sur les figures, la rampe 560 est arrangée ici entre la portion d'extrémité proximale 710 et la portion de liaison 780.La portion d'extrémité distale 720, la portion de liaison 780, la rampe 560 et la portion d'extrémité proximale 710 sont arrangées successivement, autrement dit "en série".

Dans un mode de réalisation illustré sur la figure 7, la rampe 560 et la portion de liaison 780 forment un V. Autrement dit, la rampe 560 et la portion de liaison 780 forment entre elles une zone d'inflexion. Autrement dit, le bras de fixation 500 présente un changement de direction entre la rampe 560 et la portion de liaison 780.

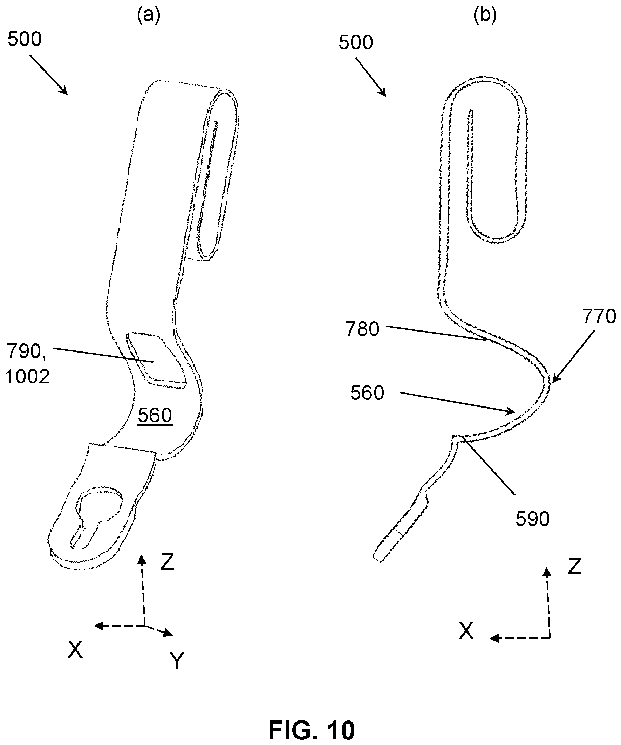

Dans une variante illustrée sur la figure 10, la rampe 560 et la portion de liaison 780 forment un U. Autrement dit, la section de collecte 595 présente une forme arrondie.

Deux variantes de la portion de liaison 780 vont être décrites par la suite.

### Variante ouverture

Dans une variante du mode de réalisation en série, illustré sur les figures 6 à 10, la zone de passage 790 est définie par une ouverture 1002 dans la portion de liaison 780. L'ouverture 1002 permet le passage de l'eau du flux d'eau F de la chasse d'eau vers la rampe 560. L'ouverture 1002 s'étend au moins jusqu'à la section de collecte 595.

L'ouverture 1002 présente une largeur transversale supérieure à 50%, notamment supérieure à 75% de la largeur transversale de la portion de liaison 780. L'ouverture présente une longueur supérieure à 50%, notamment supérieure à 75% de la largeur transversale de la portion de liaison 780.

L'ouverture 1002 présente ici une forme sensiblement rectangulaire, avec les bords arrondis. Toutefois, d'autres formes peuvent être envisagées pour permettre le passage de l'eau.

### Variante rétrécissement

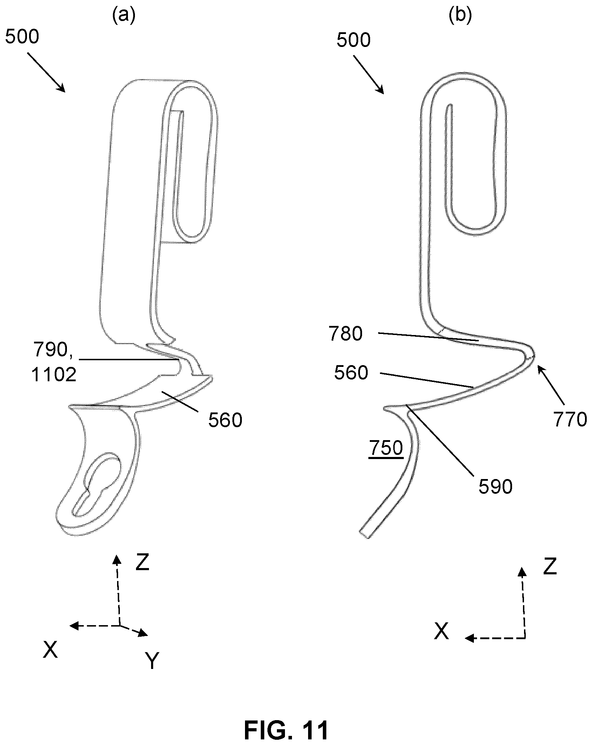

Dans une variante du mode de réalisation en série, illustrée sur la figure 11, la zone de passage 790 est définie par un rétrécissement 1102 de section au niveau de la portion de liaison 780. Le rétrécissement 1102 permet le passage de l'eau du flux d'eau F de part et d'autre du rétrécissement vers la rampe 560. En particulier, le rétrécissement 1102 est une réduction de la largeur de la portion de liaison 780 par rapport à la rampe 560. Le rétrécissement s'étend au moins jusqu'à la section de collecte 595.

Le rétrécissement 1102 présente une largeur transversale inférieure à 50%, notamment inférieure à 75% de la largeur transversale de la portion de liaison 780. Le rétrécissement présente une longueur supérieure à 50%, notamment supérieure à 75% de la largeur transversale de la portion de liaison 780.

Le rétrécissement 1102 présente ici une forme sensiblement rectiligne, centré au milieu de la portion de liaison 780. Toutefois, d'autres formes peuvent être envisagées pour permettre le passage de l'eau.

### Mode de réalisation rampe et portion de liaison "en parallèle"

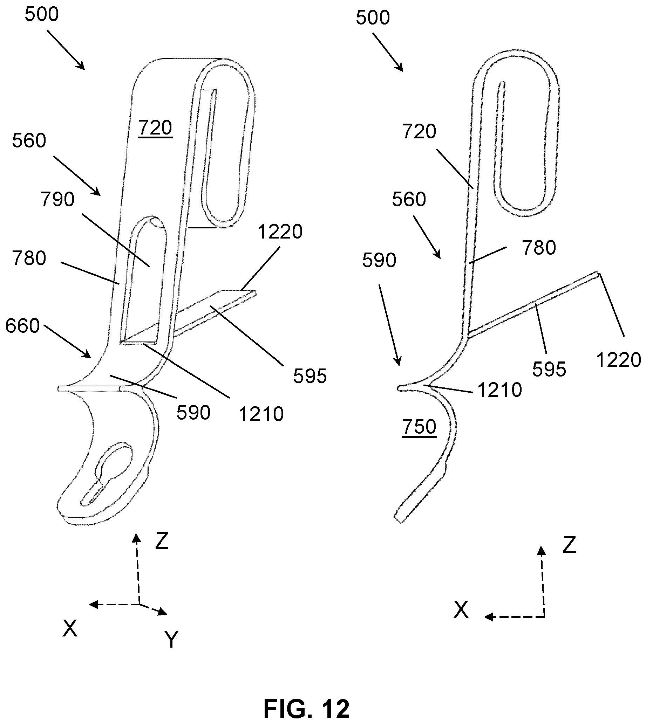

Les figures 12 à 16 présentent un mode de réalisation de l'ensemble d'analyse d'urine selon la présente description, dit "en parallèle".

Comme visible sur les figures, la portion de liaison 780 est arrangée ici entre la section de transvasement 590 de la rampe 560 et la portion d'extrémité distale 720. La section de collecte 595 de la rampe 560 s'étend à partir d'une jonction 1210 entre la portion de liaison 780 et la section de transvasement 590. En particulier, la section de collecte 595 s'étend en direction de la paroi interne 112 de la cuvette 106.

La section de collecte 595 comprend une extrémité libre 1220 du côté opposé à la jonction 1210. L'extrémité libre 1220 est en regard de la paroi interne 112 de la cuvette 106, qui est par exemple en contact de la paroi interne 112. En d'autres mots, la section de collecte 595 forme une ailette s'étendant vers la cuvette 106.

Dans ce mode de réalisation, la portion de liaison 780 et la section de collecte 595 s'étendent chacun depuis la jonction 1210, autrement dit la portion de liaison 780 et la section de collecte 595 sont "en parallèle".

### Variante en parallèle aligné

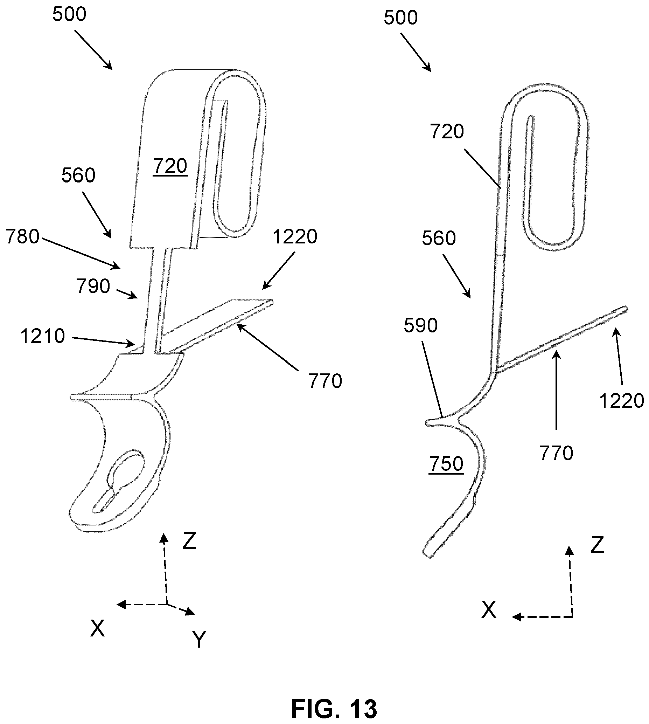

Dans une variante du mode de réalisation en parallèle illustré sur les figures 12 et 13, pour permettre le passage de l'eau de la section de collecte 595 vers la section de transvasement 590, la portion de liaison 780 comprend une zone de passage 790 (à partir de laquelle s'étend notamment la section de collecte 595 de la rampe 560, en direction opposé à la section de transvasement 590). La section de collecte 595 de la rampe 560 fait saillie depuis la zone de passage 790 en direction de la cuvette 106.

### Sous-variante ouverture

Dans une sous variante de la variante précédente, illustrée sur la figure 12, la zone de passage 790 est définie par une ouverture. L'ouverture permet le passage de l'eau depuis la section de collecte 595 à travers l'ouverture vers la section de transvasement 590. L'ouverture s'étend au moins jusqu'à la jonction 1210.

L'ouverture présente une largeur transversale supérieure à 50%, notamment supérieure à 75% de la largeur transversale de la portion de liaison 780. L'ouverture présente une longueur supérieure à 50%, notamment supérieure à 75% de la largeur transversale de la portion de liaison 780.

L'ouverture présente ici une forme d'arche. Toutefois, d'autres formes peuvent être envisagées pour permettre le passage de l'eau.

### Sous-variante rétrécissement

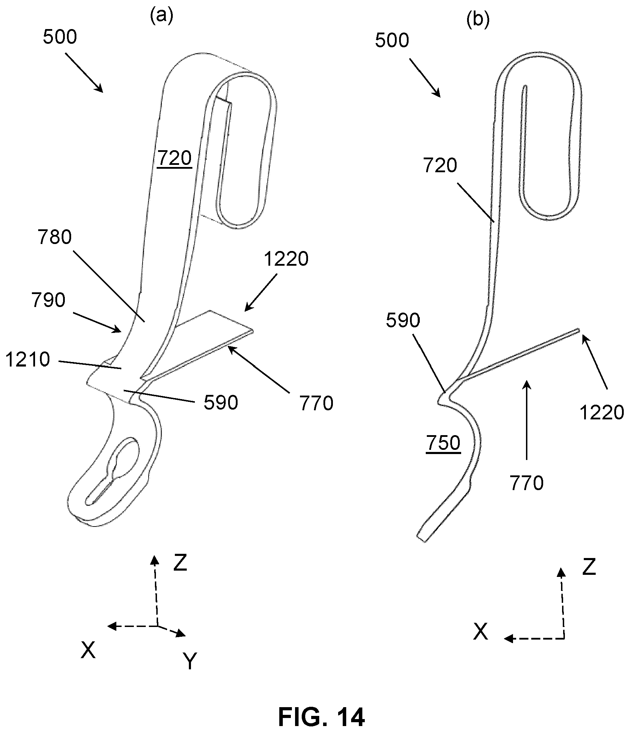

Dans un mode de réalisation illustré sur les figures 13 et 14, la zone de passage 790 est définie par un rétrécissement de section au niveau de la portion de liaison 780. Le rétrécissement permet le passage de l'eau de la chasse d'eau de part et d'autre du rétrécissement depuis la section de collecte 595 vers la section de transvasement 590. En particulier, le rétrécissement est une réduction de la largeur de la portion de liaison 780 par rapport à la rampe 560. Le rétrécissement s'étend au moins jusqu'à la jonction 1210.

Le rétrécissement présente une largeur transversale inférieure à 50%, notamment inférieure à 75% de la largeur transversale de la portion de liaison 780. Le rétrécissement présente une longueur supérieure à 50%, notamment supérieure à 75% de la largeur transversale de la portion de liaison 780.

En référence à la figure 13, le rétrécissement peut présenter une forme sensiblement rectiligne, centré au milieu de la portion de liaison 780.

Dans une variante illustrée sur la figure 14, le rétrécissement est une réduction progressive de la section transversale depuis la portion d'extrémité distale 720 jusqu'à la jonction 1210. La section de la portion de liaison 780 est donc minimale au niveau de la jonction 1210 pour permettre le passage de l'eau de part et d'autre.

Evidemment, d'autres formes peuvent être envisagées pour permettre le passage de l'eau.

### Mode de réalisation en rampe ailée

La figure 15 présente un mode de réalisation de l'ensemble d'analyse d'urine selon la présente description, dit "rampe ailée".

Comme visible sur cette figure, la rampe 560 est arrangée directement entre la portion d'extrémité proximale 710 et la portion d'extrémité distale 720. Ainsi, dans ce mode de réalisation, la portion intermédiaire 730 ne comprend pas de portion de liaison telle que définie ci-dessus.

La section de collecte 595 de la rampe 560 comprend une ou plusieurs ailettes 1510 qui s'étendent latéralement depuis une partie centrale de la rampe 560. En particulier, la section de collecte 595 comprend deux ailettes 1510 s'étendant de part et d'autre de la partie centrale de la rampe 560. De plus, les ailettes 1510 peuvent s'étendre en direction de la paroi de la cuvette, afin de venir en contact avec celle-ci.

Chaque ailette 1510 est configurée pour capter de l'eau de la chasse d'eau s'écoulant à côté du bras de fixation 500 et de le réorienter vers la section de transvasement 590 et ainsi vers le boîtier 204.

### Variante avec entonnoir

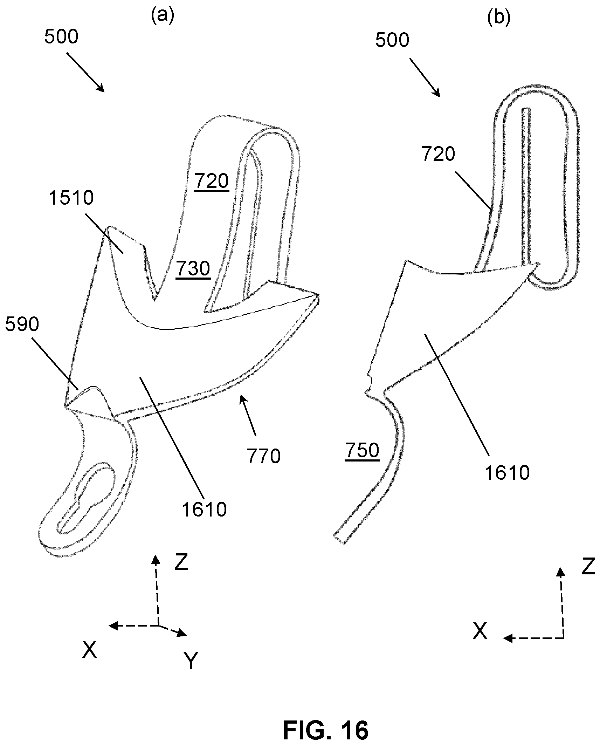

Dans une variante du mode de réalisation en rampe ailée, illustrée sur la Figure 16, la section de collecte 595 comprend un entonnoir 1610 qui s'étend autour de la partie centrale de la rampe 560. L'entonnoir peut être formé en partie par des ailettes 1510. L'entonnoir permet de canaliser le flux d'eau F et de l'orienter entièrement vers la section de transvasement 590.

### Mode de réalisation rampe unique

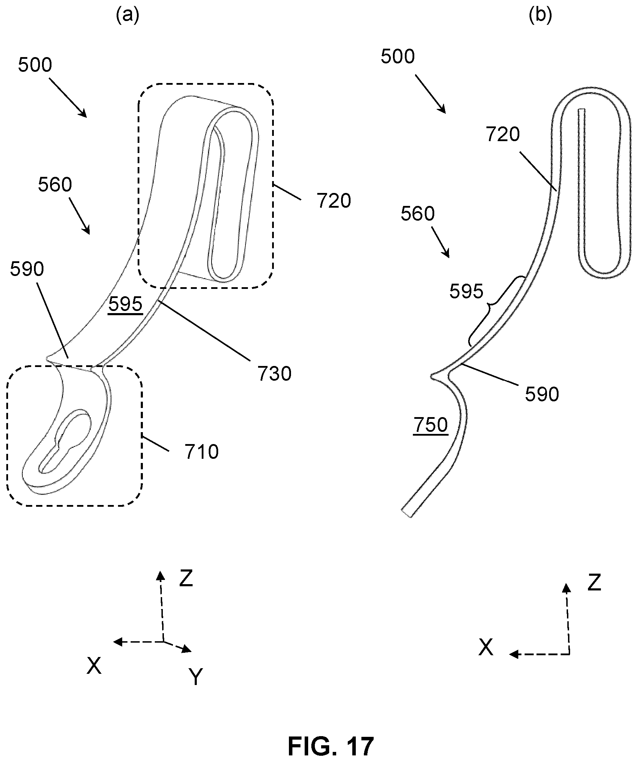

La figure 17 présente un mode de réalisation de l'ensemble d'analyse d'urine selon la présente description, dit "rampe unique".

Comme visible sur cette figure, la rampe 560 est arrangée directement entre la portion d'extrémité proximale 710 et la portion d'extrémité distale 720. Ainsi, dans ce mode de réalisation, la portion intermédiaire 730 ne comprend pas de portion de liaison telle que définie ci-dessus.

La section de collecte 595 de la rampe 560 est formée par une partie de la rampe 560 située proche, voir au contact, de la paroi interne 112 de la cuvette 106. A cet effet, la rampe 560 peut présenter une forme arrondie. En particulier, la rampe 560 peut présenter une forme convexe permettant de rapprocher la section de collecte 595 de la paroi interne 112 de la cuvette 106.

Ainsi, un flux d'eau de la chasse d'eau s'écoulant transversalement sur la paroi interne 112 est capté par la rampe 560. Selon une autre architecture de cuvette, la section de collecte 595 de la rampe 560 est configurée pour capter l'eau issue de deux flux d'eau de la chasse d'eau se réunissant au niveau du bras de fixation 500.

La section de transvasement 590 est configurée pour affleurer la face avant 220 du boîtier 204 et permettre le transfert de l'eau depuis la rampe 560 vers la face avant 220.

## Revendications

1. Ensemble pour analyse d'urine comprenant :
- un dispositif d'analyse d'urine (100) configuré pour être placé entièrement dans une cuvette (106) de toilettes et comprenant un boîtier (204), le boîtier (204) comprenant une face avant (220) destinée à recevoir un flux d'urine provenant directement d'un utilisateur urinant dans la cuvette (106) des toilettes,
- un bras de fixation (500) configuré pour positionner le dispositif d'analyse d'urine (100) entièrement dans la cuvette (106) de toilettes, le bras de fixation (500) comprenant une rampe (560) d'amenée d'eau configurée pour orienter un flux d'eau (F) provenant d'une chasse d'eau (670) de la cuvette (106) de toilettes vers la face avant (220) du boîtier (204).

2. Ensemble selon la revendication 1, dans lequel la rampe (560) s'étend dans le prolongement de la face avant (220) du boîtier (204) du dispositif d'analyse d'urine (100) ou est en surplomb de la face avant (220).

3. Ensemble selon la revendication 1 ou 2, dans lequel la rampe (560) présente au moins une ailette (1510) configurée pour s'étendre en direction de la cuvette (106) des toilettes.

4. Ensemble selon l'une quelconque des revendications précédentes, dans laquelle la rampe (560) comprend un diffuseur (910) pour l'eau de la chasse d'eau (670), le diffuseur étant configuré pour disperser l'eau du flux d'eau (F) de la rampe (560) sur la face avant (220) du boîtier (204).

5. Ensemble selon l'une quelconque des revendications précédentes, dans lequel le bras de fixation (500) comprend une portion d'extrémité proximale (710) configurée pour coopérer avec le boîtier (204).

6. Ensemble selon l'une quelconque des revendications précédentes, dans lequel la rampe (560) comprend une section de transvasement (590) à proximité du boitier et configurée pour favoriser le passage de l'eau du flux d'eau (F) de la rampe (560) vers la face avant (220) du boîtier (204).

7. Ensemble selon la revendication 6, dans lequel la section de transvasement (590) affleure la face avant (220) du boîtier (204).

8. Ensemble selon l'une quelconque des revendications précédentes, dans lequel la rampe (560) comprend une section de collecte (595) configurée pour collecter au moins une partie de l'eau de la chasse d'eau (670).

9. Ensemble selon l'une quelconque des revendications précédentes, dans lequel le bras de fixation (500) comprend en outre une portion d'extrémité distale (720) configurée pour coopérer avec la cuvette (106) de toilettes et une portion intermédiaire (730) arrangée entre la portion d'extrémité proximale (710) et la portion d'extrémité distale (720), la portion intermédiaire (730) comprenant une portion de liaison (780) et la rampe (560).

10. Ensemble selon la revendication 9, dans lequel la portion de liaison (780) comprend une zone de passage (790) permettant le passage de l'eau du flux d'eau (F).

11. Ensemble selon la revendication 10, dans lequel :
- la zone de passage (790) est définie par une ouverture (1002) dans la portion de liaison (780), ou
- la zone de passage (790) est définie par un rétrécissement (1102) de section au niveau de la portion de liaison (780).

12. Ensemble selon l'une quelconque des revendications 9 à 11, dans lequel la rampe (560) est arrangée entre la portion d'extrémité proximale (710) et la portion de liaison (780).

13. Ensemble selon l'une quelconque des revendications 9 à 11 combinée avec la revendication 6, dans lequel la portion de liaison (780) est arrangée entre la section de transvasement (590) et la portion d'extrémité distale (720).

14. Ensemble selon les revendications 8 et 13, dans lequel la section de collecte (595) de la rampe (560) s'étend à partir d'une jonction (1210) entre la portion de liaison (780) et la section de transvasement (590), la section de collecte (595) comprenant une extrémité libre (1220) du côté opposé à la jonction (1210).
